# EUROPEAN PATENT SPECIFICATION

(11) **EP 2 793 028 B1**
(45) Date of publication and mention of the grant of the patent: **20.02.2019**
(21) Application number: 11877246.6
(22) Date of filing: 21.12.2011
(51) Int. Cl.: G01N 33/68, G01N 33/53, G01N 33/574

(54) **Thioredoxin-1 as diagnostic marker for ovarian cancer**
Thioredoxin als diagnostischer Marker für Eierstockkrebs
Thiorédoxine comme marqueur diagnostique du cancer de l'ovaire

(30) Priority: 14.12.2011 KR 20110134723
(43) Date of publication of application: 22.10.2014
(73) Proprietor: Paichai University Industry-Academic Cooperation Foundation, Seo-gu, Daejeon 302-735 (KR)
(72) Inventor: KIM, IL Han, Daejeon 305-773 (KR)
(74) Representative: Dehns
(86) International application number: PCT/KR2011/009910
(87) International publication number: WO 2013/089301

(56) References cited:
- KR-A- 20100 036 243
- KR-A- 20100 104 110
- KR-A- 20110 068 695
- KR-A- 20110 126 470
- KARIHTALA PEETER ET AL: "DNA adduct 8-hydroxydeoxyguanosine, a novel putative marker of prognostic significance in ovarian carcinoma.", INTERNATIONAL JOURNAL OF GYNECOLOGICAL CANCER : OFFICIAL JOURNAL OF THE INTERNATIONAL GYNECOLOGICAL CANCER SOCIETY AUG 2009, vol. 19, no. 6, August 2009 (2009-08), pages 1047-1051, XP008175922, ISSN: 1525-1438
- PYLVAS M ET AL: "Oxidative stress-induced antioxidant enzyme expression is an early phenomenon in ovarian carcinogenesis", EUROPEAN JOURNAL OF CANCER, PERGAMON, vol. 46, no. 9, 1 June 2010 (2010-06-01), pages 1661-1667, XP027597491, ISSN: 0959-8049, DOI: 10.1016/J.EJCA.2010.02.006 [retrieved on 2010-03-04]
- IWATA YASUHIRO ET AL: "Elevated levels of thioredoxin 1 in the lungs and sera of idiopathic pulmonary fibrosis, non-specific interstitial pneumonia and cryptogenic organizing pneumonia.", INTERNAL MEDICINE (TOKYO, JAPAN) 2010, vol. 49, no. 22, 2010, pages 2393-2400, XP002738574, ISSN: 1349-7235
- TIITTO LEENA ET AL: "Expression of the thioredoxin system in interstitial lung disease.", THE JOURNAL OF PATHOLOGY NOV 2003, vol. 201, no. 3, November 2003 (2003-11), pages 363-370, XP002738575, ISSN: 0022-3417
- KIM, SW: 'Comparative proteomic analysis of advanced serous epithelial ovarian carcinoma: Predictors of chemoresistant disease' A JOURNAL OF INTEGRATIVE BIOLOGY vol. 15, no. 5, December 2008, XP055155788

## Description

### Technical Field

The present invention relates to an in vitro method for the diagnosis of ovarian cancer.

### Background Art

Thioredoxin (Trx) was discovered as a coenzyme that donates a hydrogen ion to ribonucleotide reductase, an enzyme essential for DNA synthesis in Escherichia coli. Characterized by the active site -Cys-Gly-Pro-Cys- in which the two vicinal cysteine residues interchangeably exist between the oxidated form of a disulfide bond (S-S) and the reduced form of a dithiol (-SH-SH), Trx functions as an intracellular oxidoreduction controller. Trx is a 12 kDa protein and includes thioredoxin 1 (Trx1) and thioredoxin 2 (Trx2). Trx exhibits various biological activities. For example, Trx acts as a growth factor and scavenges hydrogen peroxide which is toxic to cells. In addition, Trx has an influence on the activity of NF-κB (nuclear transcription factor κB), a transcription factor widely used in eukaryotic cells. In this regard, Trx participates in the regulation of apoptosis and tumorigenesis by cleaving disulfide bonds of oxidized proteins to turn them into their reduced state. Thanks to these biological activities, Trx has attracted intensive interest as an anti-oxidant therapeutic for the prevention of cell damage as well as an anti-cancer agent.

Ovarian cancer is a cancerous growth arising in the ovary, with the highest rate of incidence in women in their fifties to seventies. According to Korean statistics as of 2002, ovarian cancer has been diagnosed in about 1,000∼1,200 women per year and ranks second after uterine cervical cancer in the occurrence of gynecology cancer in Korea.

Epithelial ovarian cancer, which account for more than 90% of ovarian cancers, is, for the most part, diagnosed in the phase 3 or a more advanced state, with a five-year survival rate of less than 40%. In most cases like other cancers, the exact cause of ovarian cancer remains unknown. The risk of developing ovarian cancer appears to be affected by several factors. Women with a family history of ovarian cancer may have an elevated risk, which indicates the heredity nature of ovarian cancer. Persons who are diagnosed positive to a BRCA assay are 10 times more likely to be attacked by ovarian cancer than are those negative to BRCA. Therein lies the reason for conducting medical examinations early on and periodically. However, most (more than 95%) cases of ovarian cancer are diagnosed in persons with no family history. Next, patients with a personal or family history of breast cancer, endometrial cancer and/or rectal cancer may have an elevated risk of ovarian cancer. Particularly, because there is a close correlation between breast cancer and ovarian cancer, it is known that the risk of persons with a history of breast cancer to contract ovarian cancer is twice as high as those without the history, while persons with a history of ovarian cancer have a 3∼4-fold higher risk of breast cancer than those without such a history. Another factor is related to ovulation. The less the ovulation is, the lower is the risk of ovarian cancer. Pregnancy is a representative example. The risk of ovarian cancer is reduced by 10% in women who have given birth to one child and by as large as 50% in women who have undergone three childbirths, compared to those who have never given birth. After delivery, breast-feeding may also reduce the risk of certain types of ovarian cancer because it suppresses ovulation which delays menstruation. Likewise, the use of oral contraceptive pills is a protective factor because they suppress ovulation. Contemplated as risk factors are eating habits biased to the intake of a high-fat and high-protein diet, and environmental factors including asbestos and talc.

Due to its barely noticeable symptoms, approximately 65% of ovarian cancer cases are first diagnosed in a significantly developed state. It is thus recommended that women undergo a medical examination for gynecologic cancer at a regular period of one year. Generally, the diagnosis of ovarian cancer starts by a gynecologist making a physical examination of whether the ovary has become enlarged or not. If necessary, gynecologic ultrasonoscopy may be made to examine ovarian lumps. In addition, a blood test for the CA 125 tumor marker may be performed to determine whether ovarian lumps are simple cysts or malignant tumors.

Since neither ultrasonography nor the blood test is perfect, a combination of these two examinations is the most promising method of discovering ovarian cancer to date. Doctors make a decision in full consideration of all the data including the age and current and past personal and family disease histories of the patient, ultrasonoscopy and blood test results and if necessary, observation for a predetermined period of time. When a lump is suspected of being a cancer, surgery may be performed after administering a computer-aided tomography (CT) or magnetic resonance imaging (MRI). The confirmation of cancer can be made after a biopsy.

Haptoglobin (Hp) is a protein that is redundantly found in the blood, like albumin. The serum protein is synthesized mainly in hepatocytes, dermal cells, pulmonary cells and renal cells and released into the blood. It exists as a tetrameric protein consisting of two α- and two β-chains, connected by disulfide bridges. In blood plasma, haptoglobin binds free hemoglobin released from erythrocytes and thereby inhibits its oxidative activity (Wassell J.(2002) Haptoglobin: function and polymorphism. Clin Lab. 46,547-552 / Langlois M.R. & Delanghe J.R. (1996) Biological and clinical significance of haptoglobin polymorphism in humans. Clinical Chemistry 42, 1589-1600). A recent research report on proteomic research showed that haptoglobin, although in a very small amount, is released from subcutaneous and abdominal adipocytes. The protein is synthesized in the adult liver, but not in fetal livers. Haptoglobin is an acute phase protein (APP), whose plasma level rapidly increases in response to any infection or inflammatory process.

Haptoglobin exists in two allelic forms in the human population, so-called *Hp1* and *Hp2*, the latter having arisen due to the partial duplication of *Hp1* gene. Three genotypes of Hp, therefore, are found in humans: Hp1-1, Hp2-1, and Hp2-2 (Maeda N, McEvoy SM, Harris HF, Huisman TH, Smithies O. (1986) Polymorphisms in the human haptoglobin gene cluster: chromosomes with multiple haptoglobin-related (Hpr) genes. Proc Natl Acad Sci USA. 83, 73957399 / Patzelt D, Geserick G, Schroder H. (1988) The genetic haptoglobin polymorphism: relevance of paternity assessment. Electrophoresis 9, 393397). There are two Hp alpha chain (Hpα) isoforms: alpha 1 and alpha 2. These alpha proteins exist as position variants in various species (Sadrzadeh SM, Bozorgmehr J. (2004) Haptoglobin phenotypes in health and disorders. Am J Clin Pathol. 121 Suppl: S97-104).

Among the diseases associated with the gene for the protein are diabetic nephropathy and Crohn's disease. As haptoglobin is indeed an acute-phase protein, any inflammatory process such as infection, extreme stress, burns, major crush injury, allergy, etc. may increase the levels of haptoglobin in the plasma. A recent report demonstrates that patients with cancer including ovarian cancer have elevated levels of plasma haptoglobin.

Considering such reports, haptoglobin is not suitable for use as a diagnostic marker for pneumonia due to the lack of specificity. Hp is useful as a cancer marker because its plasma level increases in various cancers including ovarian cancer, but it cannot be used to discriminate between cancer and pneumonia. Accordingly, there is a need for a marker by which pneumonia can be specifically diagnosed and prognosticated after having been distinguished from cancer. Likewise, because the expression level of most cancer markers increases in response to inflammation, there is a pressing need for a cancer marker the plasma level of which increases in response to cancer, but not in response to inflammation.

P. Karithala et al (2009), Int. Gynecol. Cancer 19(6), 1047-1051 shows that thioredoxin is overexpressed in ovarian carcinomes. M. Pylväs et al (2010) Eur. J. Cancer 46(9), 1661-1667 show that the level of thioredoxin in tumor tissue samples can be used as a tool for the differential diagnosis between benign and borderline ovarian tumors. Y. Iwata et al (2010), Internal Medicine 49(22), 2393-2400 shows that thioredoxin is overexpressed in serum from patients with idiopathic interstitial pneumonia.

### Disclosure

### Technical Problem

Culminating in the present invention, intensive and thorough research into the simple and selective diagnosis of ovarian cancer and pneumonia using the blood, which is a relatively easily obtainable specimen, aiming at overcoming the problems encountered in the prior art, resulted in the finding that healthy persons and patients affected with the diseases have different plasma levels of thioredoxin 1.

### Technical Solution

It is an object of the present invention to provide an in vitro method for the diagnosis of ovarian cancer as defined in the appended claims.

### Advantageous Effects

Because blood is employed as a specimen and it is relatively easy to sample, the diagnosis method of ovarian cancer and/or pneumonia in accordance with the present invention is very simple and does not subject patients to a load compared to conventional methods that are directed to a biopsy. In addition, the method of the present invention is useful in the early diagnosis of ovarian cancer thanks to its high diagnostic sensitivity and selectivity. Concurrently, thioredoxin 1 can be used as a diagnostic marker for pneumonia, which is characterized by a decreased serum level, with high selectivity for pneumonia, thereby readily discriminating pneumonia from cancer and non-cancer diseases.

### Description of Drawings

FIG. 1 is a diagram showing the correlation of serum Trx1 levels of the normal male and female control, determined by ELISA, with age.
FIG. 2 is a diagram showing the correlation of serum Trx1 levels of ovarian cancer patients, determined by ELISA, with age.
FIG. 3 shows graphs in which serum Trx1 levels are plotted against the progress of ovarian cancer.
FIG. 4 is a diagram showing serum Trx1 levels, determined by ELISA, in patients with ovarian cancer and inflammatory diseases including community acquired pneumonia (CAP), arthritis, rheumatoid arthritis, atopic dermatitis, and cardiovascular disease.
FIG. 5 shows diagrams in which serum Trx1 and haptoglobin (Hp) levels of pneumonia patients are compared.
FIG. 6 shows the correlation of serum levels of Trx1 and Hp, a pneumonia marker, with the onset of pneumonia as measured in normal male and female controls and in pneumonia patients.

### Best Mode

The present inventors have done studies on the diagnosis of ovarian cancer that are simplistic and selective and found that there is a difference in the plasma level of thioredoxin between patients with the disease and healthy persons, which led to the present invention.

A detailed description will be given of the present invention, below.

According to the present disclosure the concentration of thioredoxin 1 in a specimen can be measured using an antigen-antibody reaction, thereby creating information necessary for the diagnosis of ovarian cancer and/or pneumonia. No limitations are imparted to the sample. The specimen is selected from the group consisting of a tissue, an extract, a cell lysate, whole blood, plasma, serum, ocular fluid, cerebrospinal fluid, sweat, urine, milk, ascites, synovial fluid, and peritoneal fluid.

Thioredoxin 1 was found to be present in significantly higher concentrations in the blood of ovarian cancer patients than in the blood of normal persons, as measured by ELISA. In addition, the plasma level of thioredoxin 1 in patients with ovarian cancer was measured to be significantly lower than that in patients with non-cancer inflammatory diseases and cardiovascular diseases. Further, the plasma level of thioredoxin was observed to increase with the progress of cancer. Significantly higher plasma levels of thioredoxin 1 were detected even in patients with early ovarian cancer than in normal persons. Ovarian cancer patients who were determined to be negative to CA125, a conventional ovarian cancer marker, were observed to have plasma thioredoxin 1 levels as high as those of CA125-positive patients. Therefore, thioredoxin 1 can be utilized as a diagnostic marker for ovarian cancer.

In addition, patients with non-cancer, inflammatory diseases had lower plasma thioredoxin 1 levels than normal persons. Particularly, pneumonia patients were found to have thioredoxin 1 concentrations significantly lower than those of normal persons, showing that thioredoxin 1 allows non-cancer inflammatory diseases to be discriminated in an excellent manner from other diseases.

Because blood which is relatively easy to be sampled is used as the specimen, as described above, the diagnosis method of ovarian cancer in accordance with the present disclosure is very simple without imparting a load to patients compared to conventional methods that are directed to biopsy. In addition, this method is useful for the early diagnosis of ovarian cancer thanks to the high diagnostic sensitivity and selectivity thereof.

A better understanding of the present invention may be obtained through the following examples which are set forth to illustrate, but are not to be construed as limiting the present invention.

### EXAMPLES

### Subjects

All sera of normal persons (control) and patients were obtained from white Caucasians. The sera and the clinical information thereof were provided from Asterand (U. S. A.) and Bioserve (U. S. A.) as summarized in Tables 1 and 2, below.

**TABLE 1**

| **Characteristics** | **Number of samples** |
|---|---|
| **Normal control:** Caucasian, White | |
| Male (Mean ±SD age, years) | 50 (44.54±14.85) |
| Female (Mean±SD age, years) | 49 (44.18±14.58) |

| **Ovarian cancer patient:** Caucasian, White | |
|---|---|
| Female (Mean±SD age, years) | 112 (57.63±11.69) |

| **Cancer Stage** | |
|---|---|
| Stage I | 21 |
| Stage II | 32 |
| Stage III | 43 |
| Stage IV | 12 |
| N/A | 4 |

| **Cancer Grade** | |
|---|---|
| Grade 1: Well differenciated | 8 |
| Grade 2: Moderately differentiated | 31 |
| Grade 3: Poorly differenciated | 28 |
| N/A | 45 |

| **CA125 Test** | |
|---|---|
| Positive (>CA125 35U/ml) | 30 |
| Negative (<CA125 35U/ml) | 17 |
| N/A | 65 |

**TABLE 2**

| **Type of Disease** | **Number of samples** (Age, Mean±SD) |
|---|---|
| **CAP (Pneumonia)**: Caucasian, White | **80** (67.03±16.79) |
| Male | 40 (67.35±16.58) |
| Female | 40 (66.7±17.19) |

| **Arthritis**: Caucasian, White | **60** (61.58±10.55) |
|---|---|
| Male | 30 (60±10.77) |
| Female | 30 (50.47±13.09) |

| **Cardiovasicular Disease:** Caucasian, White | **60** (64.80±10.86) |
|---|---|
| Male | 30 (64.63±10.64) |
| Female | 30 (64.97±11.26) |

| **Rheumatoid Arth.:** Caucasian, White | **14** (66.43±13.15) |
|---|---|
| Male | 3 (65.33±10.97) |
| Female | 11 (66.73±14.16) |

| **Atopic Dermatitis**: Caucasian, White | **18** (59.78±17.48) |
|---|---|
| Male | 7 (55.86±23.44) |
| Female | 11 (62.27±13.12) |

### ELISA Assay for Plasma Protein Level

ELISA (Enzyme-linked immunosorbent assay) was performed to quantitatively analyze blood proteins.

Serum levels of proteins were determined using an ELISA kit using antibodies of interest (Express ELISA kit (rabbit), GenScript). In this regard, antibodies to thioredoxin 1 (Trx1) and haptoglobin (HP) were obtained by injecting purified proteins into rabbits to form antisera and purifying the antisera on a protein A column.

Protein levels were determined by absorbance at 450 nm in triplicate, and mean values of three measurements were given. For statistical analysis, the software GraphPad Prisn (ver. 5.04) (GraphPad Software) was used.

### EXAMPLE 1: Level of Trx1 in Ovarian Cancer Patients

Difference in blood Trx1 level between normal controls and ovarian cancer patients was examined.

In detail, serum Trx1 levels were measured in 49 normal females distributed over a wide range of age (age distribution: ages of 41 to 85) and 112 ovarian cancer patients and subjected to ROC (receiver operating characteristic) curve plotting.

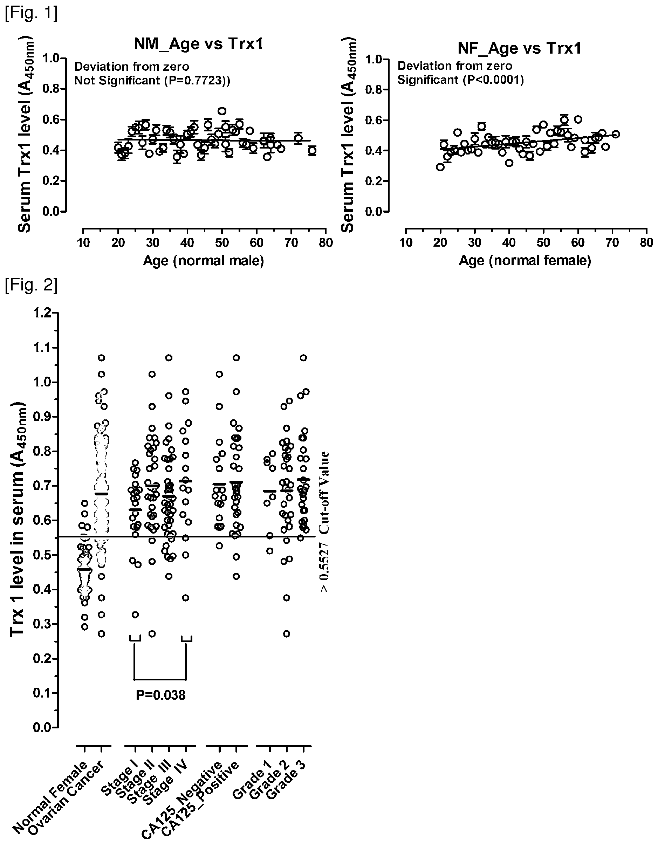

Concentration measurements are graphically shown in FIG. 1 and statistically summarized in Table 3. AUC (Area under the ROC Curve) values calculated by the partial integration of the ROC curve, and sensitivity and specificity values are summarized in Table 4, below.

**TABLE 3**

| | NF | OC | Stage I | Stage II | Stage III | Stage IV | CA125 Negative | CA125 Positive | Grade 1 | Grade 2 | Grade 3 |
|---|---|---|---|---|---|---|---|---|---|---|---|
| Number of values | 49 | 112 | 21 | 32 | 43 | 16 | 17 | 30 | 8 | 31 | 28 |
| Mean | 0.459 | 0.677 | 0.630 | 0.700 | 0.670 | 0.714 | 0.705 | 0.711 | 0.684 | 0.686 | 0.718 |
| Std. Deviation | 0.072 | 0.136 | 0.107 | 0.144 | 0.129 | 0.164 | 0.132 | 0.142 | 0.107 | 0.153 | 0.133 |
| Std. Error | 0.0103 | 0.0129 | 0.0232 | 0.0254 | 0.0196 | 0.0411 | 0.032 | 0.0259 | 0.0377 | 0.0274 | 0.0251 |

**TABLE 4**

| | OC (total) | Ovarian Cancer (OC) Stage | | | | Ovarian Cancer Grade | | |
|---|---|---|---|---|---|---|---|---|
| | | I | II | III | IV | 1 | 2 | 3 |
| AUC ±SE | 0.93 ±0.0203 | 0.904 ±0.0507 | 0.941 ±0.0338 | 0.942 ±0.0226 | 0.913 ±0.0598 | 0.964 ±0.0283 | 0.903 ±0.0454 | 0.98 ±0.0115 |
| Sensitivity (%) | 85.7 | 85.7 | 90.6 | 83.7 | 81.2 | 100 | 83.9 | 96.4 |
| Specificity (%) | 91.8 | 91.8 | 91.8 | 91.8 | 95.9 | 79.6 | 91.8 | 91.8 |

As can be seen in FIG. 1 and Table 3, the mean concentration values of Trx1 in the female normal control (NF) and the ovarian cancer group (OC) were 0.459 and 0.677, respectively, showing that the serum level of Trx1 increases by about 48% in ovarian cancer patients. Meanwhile, thioredoxin 1 was measured at substantially the same concentrations in groups both negative (0.705) and positive (0.711) to CA125, a conventional ovarian cancer marker.

The experimental data in which the serum levels of Trx1 in both CA125-negative and positive ovarian cancer patients are much higher those in the normal control indicate that Trx1 is much more efficient as a diagnostic maker for ovarian cancer than is the conventional marker CA125.

In addition, patients with early ovarian cancer, which correspond to Stage I and Grade 1 of Table 1, were found to have concentrations of 0.630 and 0.684, which are 37% and 49% higher, respectively, than the normal concentration 0.459 (Table 4).

As shown in Table 4, AUC values of Stage I and Grade 1 patient groups are 0.904 and 0.964, respectively, which are similar to the AUC value, 0.93, measured for all of the ovarian cancer patients. In addition, FIG. 2 shows that serum Trx1 levels increase with the progress of cancer. In all cases, AUC values, which are indicative of the probability of cancer discovery, were 0.9 or higher, the specificity that is indicative of discrimination from normal groups was about 80%, and the sensitivity that is indicative of detecting cancer patients was more than 81%.

### EXAMPLE 2: Serum Level of Trx1 in Patients with Ovarian Cancer and Non-Cancer Diseases

An examination was made of the difference in serum Trx1 level between patients with ovarian cancer and non-cancer patients.

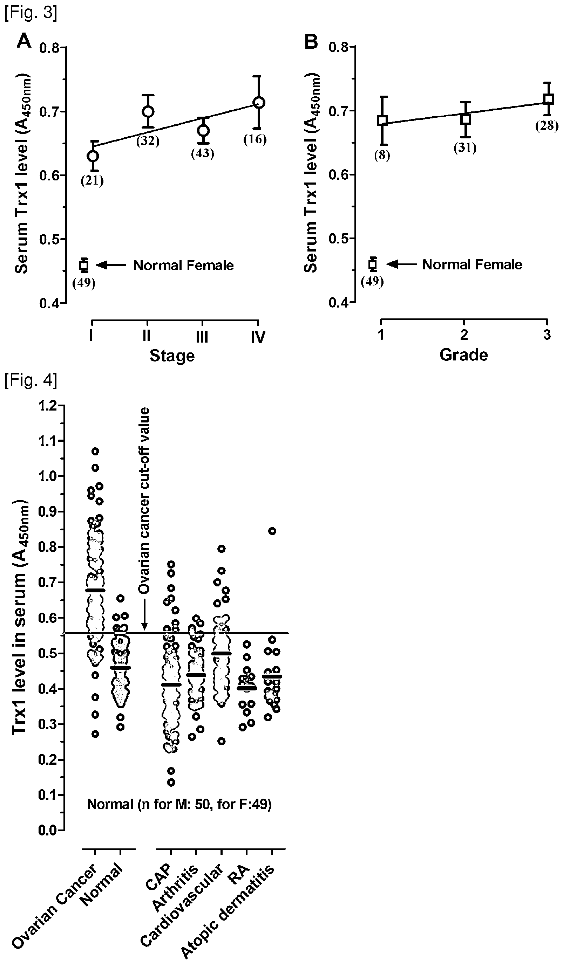

Serum Trx1 levels were measured in patients with ovarian cancer (OC), pneumonia (CAP), arthritis, cardiovascular disease, rheumatoid arthritis and atopic dermatitis as well as in normal male and female controls, using ELISA. The results are shown in FIG. 4 statistics of the measurements are summarized in Table 5. AUC, Sensitivity, Specificity and Cut-off values, obtained by ROC curve analysis of the measurements, are given in Table 6, below.

**TABLE 5**

| | Normal | CAP | Arthritis | Cardiovasicular | RA | Atopic dermatitis |
|---|---|---|---|---|---|---|
| Number of values | 99 | 80 | 60 | 60 | 14 | 18 |
| Mean | 0.459 | 0.411 | 0.438 | 0.499 | 0.402 | 0.435 |
| Std. Deviation | 0.0678 | 0.119 | 0.0691 | 0.0999 | 0.0672 | 0.118 |
| Std. Error | 0.00682 | 0.0133 | 0.00892 | 0.0129 | 0.0180 | 0.0279 |

**TABLE 6**

| | CAP (Pneumonia) | Arthritis | Cardiovasicular Disease | RA | Atopic dermatitis | Normal control |
|---|---|---|---|---|---|---|
| AUC ±SE | 0.928 ±0.02 | 0.948 ±0.0168 | 0.866 ±0.0292 | 0.968 ±0.015 | 0.913 ±0.0509 | 0.93 ±0.0183 |
| Sensitivity(%) | 87.5 | 85.7 | 82.1 | 89.3 | 88.4 | 82.1 |
| Specificity(%) | 88.7 | 93.3 | 81.7 | 100 | 94.4 | 97 |
| Cut-off value (A450nm) | 0.5455 | 0.5539 | 0.5681 | 0.5257 | 0.5383 | 0.5527 |

As seen in Table 5, serum Trx1 levels of normal male and female controls and non-cancer patients groups were distributed over the range of from 0.4 to 0.5, which were significantly lower than the measurement of the ovarian cancer group, 0.667 (Table 3). As can be seen in FIG. 4, almost all measurements of the non-cancer groups were below 0.5527, the cut-off value for discriminating the normal control from the ovarian cancer group. In order to confirm this result, ROC curve analysis was performed on the measurements of the ovarian cancer group, with the Trx1 measurements of the normal male and female controls and the non-cancer groups used as references. As can be gleaned from the results of Table 6, ROC analysis results were generally similar between the control groups and the non-cancer groups, indicating that Trx1 may be used as a promising ovarian cancer marker capable of clearly discriminating ovarian cancer patients whether they have been affected with non-cancer diseases. As for the cut-off values, all of them fall within the range of 0.54-0.57, which demonstrates the superiority of Trx1 as an ovarian cancer marker.

Taken together, the data obtained above indicate that Trx1 can discriminate ovarian cancer groups from normal female controls at a probability of about as high as 93% and can be used as an ovarian cancer marker superior in sensitivity and selectivity for ovarian cancer whether or not the patients are affected by non-cancer diseases.

### EXAMPLE 3: Serum Trx1 Level of Pneumonia Patients (comparative example)

In order to demonstrate the utility of Trx1 as an ovarian cancer marker, sera from patients suffering from pneumonia, an inflammatory disease of the relatively large organ of the lung, were analyzed for Trx1 levels. The reason is that because the intracellular synthesis of Trx1 is increased in response to oxidative stress, serum Trx levels may also be increased in response to inflammatory diseases such as pneumonia; if so, the use of Trx1 as an ovarian cancer marker would be significantly restricted.

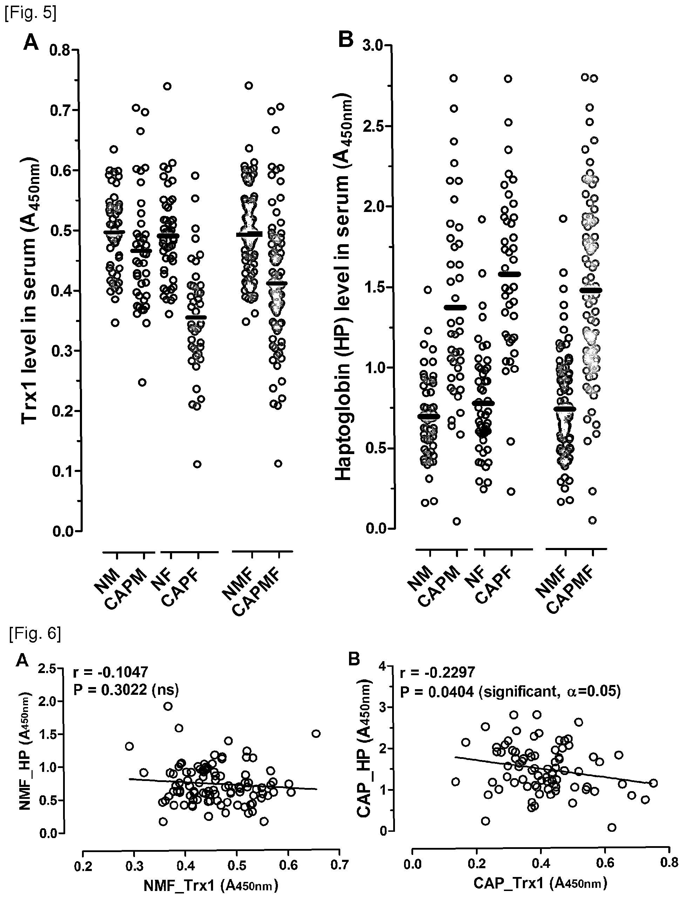

As shown in FIG. 4, serum Trx1 levels were at remarkably lower values in pneumonia patients than in the other non-cancer disease patients. Thus, an examination was made of the significance of Trx1 as a diagnostic marker over haptoglobin (HP), currently used as a marker. In this regard, serum levels of two proteins (Trx1 and Hp) were measured in normal controls of men (n=50) and women (n=49) and pneumonia patients of men (n=40) and women (n=40) (CAP_M, CAP_F. CAP_MF). The results are shown in FIG. 5. To assay the two proteins (Trx1 and Hp) for utility as lung cancer markers, the measurements were subjected ROC curve plotting and the results are summarized in Table 7, below.

**TABLE 7**

| | Thioredoxin 1 (Trx1) | | | Haptoglobin (HP) | | |
|---|---|---|---|---|---|---|
| | CAP_M | CAP_F | CAP_MF | CAP_M | CAP_F | CAP_MF |
| AUC ±SE | 0.638 ±0.0621 | 0.877 ±0.0395 | 0.756 ±0.0385 | 0.865 ±0.0398 | 0.905 ±0.0366 | 0.882 ±0.0271 |
| Sensitivity (%) | 65 | 72.5 | 51.2 | 72.5 | 82.5 | 81.2 |
| Specificity (%) | 64 | 89.8 | 92.9 | 88 | 89.8 | 83.8 |
| Cut-off value (A450nm) | 0.4794 | 0.3964 | 0.3969 | 0.9583 | 1.1357 | 0.9953 |

For the male and female pneumonia group over the normal male and female control, as can be seen in Table 7, AUC values of Trx1 and Hp were measured to be 0.756 and 0.882, with a sensitivity of 52.2% and 81.2% and a specificity of 92.9% and 83.8%, respectively. This data shows that Trx1 and Hp are superior in specificity and selectivity, respectively.

As is understood from the data, Hp is a good marker for both male and female patients with pneumonia while Trx1 exhibits a function similar to Hp in female patients. The characteristic difference between Hp and Trx1 is that the pneumonia group has a higher serum Hp level but a lower serum Trx1 level than does the normal control. To confirm these contrary results, the correlation between the Trx1 measurements and the Hp measurements of the pneumonia group was analyzed, and the results are shown in FIG. 6.

The Trx1 measurements were found to be inversely proportion to the Hp measurements, as seen in FIG. 6. Further, this relation was more surely established in the female pneumonia group (FIG. 5 and Table 7).

On the basis of the results obtained above, Trx1 may be used as a diagnostic marker for pneumonia which is characterized by having a lower serum level compared to a control. Thanks to this characteristic, Trx1 has an advantage over Hp in selecting pneumonia patients. This is because serum Hp levels increase in patients with various cancers including ovarian cancer and non-cancer disease as well as pneumonia, without specificity.

Consequently, Trx1 can be used not only as an ovarian cancer marker because it is capable of discriminating ovarian cancer patients from normal females at a probability of about 93%, but also a diagnostic marker for pneumonia. Unlike Hp, the serum level of Trx1 significantly increases in ovarian cancer patients, compared to a control, with excellent sensitivity and selectivity for ovarian cancer whether the patients have been affected with non-cancer diseases or not.

### Industrial Applicability

As described hitherto, the present invention provides a method for diagnosing ovarian cancer by measuring serum Trx1 levels.

## Claims

1. An in vitro method for the diagnosis of ovarian cancer in a patient, said method comprising measuring the level of thioredoxin 1 in a specimen of whole blood, plasma or serum from the patient.

2. The in vitro method of claim 1, wherein the specimen is serum.

3. The in vitro method of claim 1 or claim 2, wherein the level of thioredoxin 1 is measured using an antibody that specifically binds to thioredoxin 1.

## Patentansprüche

1. In-vitro-Verfahren für die Diagnose von Eierstockkrebs in einem Patienten, wobei das Verfahren ein Messen des Thioredoxin-1-Spiegels in einer Vollblut-, Plasma- oder Serumprobe vom Patienten umfasst.

2. In-vitro-Verfahren nach Anspruch 1, wobei die Probe Serum ist.

3. In-vitro-Verfahren nach Anspruch 1 oder Anspruch 2, wobei der Thioredoxin-1-Spiegel unter Verwendung eines Antikörpers gemessen wird, der sich spezifisch an Thioredoxin-1 bindet.

## Revendications

1. Procédé in vitro pour le diagnostic d'un cancer ovarien chez une patiente, ledit procédé comprenant la mesure du niveau de thiorédoxine 1 dans un échantillon de sang total, de plasma ou de sérum provenant de la patiente.

2. Procédé in vitro selon la revendication 1, dans lequel l'échantillon est du sérum.

3. Procédé in vitro selon la revendication 1 ou la revendication 2, dans lequel le niveau de thiorédoxine 1 est mesuré en utilisant un anticorps qui se lie de manière spécifique à la thiorédoxine 1.
